# EUROPEAN PATENT SPECIFICATION

(11) **EP 4 440 688 B1**
(45) Date of publication and mention of the grant of the patent: **13.08.2025**
(21) Application number: 22821462.3
(22) Date of filing: 22.11.2022
(51) Int. Cl.: A61N 1/375, A61N 1/39, A61N 1/365, A61N 1/37, A61N 1/372

(54) **IMPLANTABLE MEDICAL DEVICE FOR PROVIDING A DIAGNOSTIC AND/OR THERAPEUTIC CARDIAC FUNCTION WITHIN A PATIENT**
IMPLANTIERBARE MEDIZINISCHE VORRICHTUNG ZUR BEREITSTELLUNG EINER DIAGNOSTISCHEN UND/ODER THERAPEUTISCHEN HERZFUNKTION IN EINEM PATIENTEN
DISPOSITIF MÉDICAL IMPLANTABLE POUR LA FOURNITURE D'UN DIAGNOSTIC ET/OU D'UNE FONCTION CARDIAQUE THÉRAPEUTIQUE CHEZ UN PATIENT

(30) Priority: 01.12.2021 EP 21211619
(43) Date of publication of application: 09.10.2024
(73) Proprietor: BIOTRONIK SE & Co. KG, 12359 Berlin (DE)
(72) Inventor: DOERR, Thomas, 12437 Berlin (DE); LANG, Volker, 12161 Berlin (DE); DIEM, Bjoern Henrik, 14197 Berlin (DE); WIST, Dominic, 10318 Berlin (DE); BECKER, Frank, 10409 Berlin (DE); KRUEGER, Daniel, 15741 Bestensee (DE); HENNIG, Carsten, 13591 Berlin (DE)
(74) Representative: Biotronik Corporate Services SE
(86) International application number: PCT/EP2022/082790
(87) International publication number: WO 2023/099282

(56) References cited:
- US-A1- 2018 043 158
- US-A1- 2019 111 270
- US-A1- 2019 329 045
- US-A1- 2020 215 340
- US-B1- 9 168 380
- US-B2- 9 675 811

## Description

The instant invention generally relates to an implantable medical device for providing a diagnostic and/or therapeutic cardiac function within a patient, to an implantable therapy system.

Generally, an implantable medical device comprises a sensing arrangement for sensing electrocardiogram signals, a communication circuitry for data communication with a device external to the patient, and a processing circuitry for processing sensed electrocardiogram signals.

An implantable medical device of the type concerned herein for example may be an implantable cardioverter defibrillator (ICD), which in particular may be configured for a non-transvenous implantation external to the patient's heart.

Within an implantable therapy system, multiple implantable medical devices may be implanted in a patient, for example an implantable medical device in the shape of an implantable cardioverter defibrillator together with a pacemaker device, such as a leadless pacemaker. Devices of the implantable therapy system herein shall communicate with an external device, for example within a home monitoring system, such that information concerning devices of the implantable therapy system is communicated to the external device and, via the external device, may be relayed e.g. to a home monitoring service center for assessment e.g. by a physician.

Within an implantable therapy system, some devices may be implanted deeper in a patient than others. In addition, some devices, such as for example a leadless pacemaker device, may be small in construction and hence may comprise only a comparatively small battery comprising a limited energy storage capacity, such that those devices shall operate in a particularly energy-efficient manner.

In order to allow for a communication between devices of an implantable therapy system, approaches exist for establishing an intra-body communication network. Intra-body communication techniques however require the implantable medical devices to interact in a defined manner, posing rather strict compatibility restrictions on the implantable medical devices.

In other approaches, a communication with implantable medical devices implanted deep in a patient may involve low frequency communication techniques, for example employing a coil telemetry communication. This however requires the use of a reading device which must be approached externally to the patient in order to establish a data transmission, making a communication potentially unreliable and cumbersome for a user.

EP 2 327 609 B1 describes an acoustic communication link in between implanted medical devices for exchanging information in between the implanted medical devices. The acoustic communication link is established to permit wireless communication between the implanted medical devices, wherein transmission parameters may be adapted, such as a sensitivity and a carrier frequency, in order to improve an existing communication link.

US 2010/0022836 A1 discloses multidirectional transmitters for in-body devices, such as implantable devices.

US 9 168 380 B1 discloses a medical device system including an intracardiac pacemaker that is configured to receive by an implantable medical device sensing module a first cardiac signal using a first pair of electrodes implanted outside the cardiovascular system and identify a P-wave from the first cardiac signal. The system transmits a wireless trigger signal to the intracardiac pacemaker in response to identifying the P-wave. The intracardiac pacemaker delivers a pacing therapy in response to the trigger signal.

US 9 675 811 B2 discloses a cardiac rhythm management system including a first implantable device such as a defibrillator and a second implantable device such as a leadless cardiac pacemaker. A programmer is configured to receive and display heart data emanating from the implantable defibrillator and from the leadless cardiac pacemaker. The heart data emanating from the leadless cardiac pacemaker is displayed in temporal alignment with the heart data emanating from the implantable defibrillator.

US 2020 215 340 A1 discloses an implantable system including an atrial leadless pacemaker (aLP) and a ventricular leadless pacemaker (vLP), and methods for use therewith, that are configured or used to terminate a pacemaker mediated tachycardia (PMT). One of the aLP or the vLP detects a PMT and informs the other one. The aLP initiates a PMT PA interval that is shorter than a PA interval that the aLP would otherwise use for atrial pacing if a PMT was not detected. The vLP initiates a PMT PV interval that is longer than the PMT PA interval. If an intrinsic atrial or ventricular event is detected before PMT PA interval or the PMT PV interval expires, then these intervals will be terminated, otherwise an atrial chamber will be paced if the PMT PA interval expires, and/or a ventricular chamber will be paced if the PMT PV interval expires. This should have the effect of terminating the PMT.

US 2018 004 3158 A1 discloses systems and methods for monitoring patients with cardiac diseases. A system may include an accelerometer sensor for sensing an epicardial or endocardial acceleration (EA) signal. The accelerometer may be included in a leadless or a lead-based medical device. The system may additionally include a companion device in communication with the medical device via a wireless communication link. The companion device may sense a cardiac signal such as a heart sound signal, and may use the cardiac signal and the EA signal transmitted from the medical device to generate a diastolic function indicator indicating the diastolic function of the heart. The system may provide the diastolic function indicator to a user or a process, or to detect worsening of heart failure based on the diastolic function indicator.

US 2019 011 1270 A1 deals with pacing of cardiac tissue, and more particularly with adjusting delivery of His bundle or bundle branch pacing in a cardiac pacing system to achieve synchronized ventricular activation. Bundle pacing may be delivered in response to determining whether the QRS parameter or activation interval is greater than a threshold. A set of AV delays may be generated, and an optimal AV delay may be selected from the stored set of AV delays. His-bundle or bundle-branch pacing may be selectively delivered based on RV or LV activation time. Pacing may also be adjusted based on dyssynchrony detected or the type of bundle branch block pattern detected.

US 2019 329 045 A1 discloses an implantable medical device system including an extracardiac sensing device and an intracardiac pacemaker. The sensing device senses a P-wave attendant to an atrial depolarization of the heart via housing-based electrodes carried by the sensing device when the sensing device is implanted outside the cardiovascular system and sends a trigger signal to the intracardiac pacemaker in response to sensing the P-wave. The intracardiac pacemaker detects the trigger signal and schedules a ventricular pacing pulse in response to the detected trigger signal.

It is an object of the instant invention to provide an implantable medical device and an implantable therapy system for operating an implantable medical device which allow for an easy and reliable monitoring and information transmission concerning a device which may be implanted deep in a patient.

This object is achieved by means of an implantable medical device comprising the features of claim 1. Aspects, embodiments and examples disclosed herein which do not fall within the scope of the appended claims do not form part of the invention, and are merely provided for illustrative purposes.

In one aspect, an implantable medical device for providing a diagnostic and/or therapeutic cardiac function within a patient, comprises: a sensing arrangement for sensing electrocardiogram signals; a communication circuitry for data communication with a device external to the patient; and a processing circuitry for processing sensed electrocardiogram signals, wherein the processing circuitry is configured to identify the conduction of a threshold test measurement by an implantable pacemaker device and to cause a transmission of information concerning said threshold test measurement to the device external to the patient.

The implantable medical device may in particular be an implantable non-transvenous defibrillator device configured to emit a shock pulse for achieving a defibrillation. An implantable non-transvenous defibrillator device, also denoted as non-transvenous implantable cardioverter defibrillator (in short non-transvenous ICD), may serve for monitoring and treating potentially life-threatening arrhythmias of a patient's heart. The implantable non-transvenous defibrillator device is configured for non-transvenous implantation, that is an implantation such that no electrode leads transvenously are implanted within the heart of a (human or animal) patient. The implantable non-transvenous defibrillator device hence is to be implanted in a patient such that a generator and an electrode arrangement, for example a shock electrode placed on a lead connected to the generator, are implanted extracardially and do not reach into the heart of the patient, that is into the right or left ventricle or the right or left atrium.

In one embodiment, the implantable non-transvenous defibrillator device comprises a lead to be implanted extracardially and a shock electrode arranged on the lead. The shock electrode herein, in an implanted state of the defibrillator device, is placed outside of the heart of the patient, for example in the region of the sternum of the patient, such that a shock pulse for achieving a defibrillation is generated outside of the heart. Generally, the implantable non-transvenous defibrillator device does not comprise any portions which extend transvenously into the heart, but the defibrillator device is configured to achieve a sensing and emission of signals outside of the heart.

In another embodiment, the implantable medical device is an implantable monitoring device, such as a rhythm monitoring device, for example configured for a subcutaneous implantation in a patient. In yet another embodiment, the implantable medical device is a recording device, such as an implantable loop recorder, or an implantable sensor device.

The implantable medical device comprises a sensing arrangement for sensing electrocardiogram signals. The sensing arrangement may for example comprise one or multiple pairs of electrode poles via which electrocardiogram signals may be sensed and forwarded to processing circuitry operatively connected to the sensing arrangement.

The processing circuitry is configured to process sensed electrocardiogram signals, wherein the processing circuitry may for example be generally configured to identify, within the sensed electrocardiogram signals, stimulation events caused by an implantable pacemaker device. The implantable medical device hence is programmed to derive, from sensed electrocardiogram signals, information relating to another implantable device, namely an implantable pacemaker device, which is separate and independent from the implantable medical device.

The implantable pacemaker device in particular may be a leadless pacemaker device which is directly implanted into the heart of the patient, for example into the right or left ventricle, and which does not comprise leads carrying electrodes.

Specifically, the processing circuitry of the implantable medical device is configured to identify the conduction of a threshold test measurement by an implantable pacemaker device, e.g. by identifying stimulation events based on sensed electrocardiogram signals relating to a threshold test measurement conducted by an implantable pacemaker device, and to cause a transmission of information concerning the threshold test measurement to the device external to the patient. The implantable medical device hence is operative to obtain information relating to a threshold test measurement conducted by an implantable pacemaker device and to communicate such information to the external device.

Generally, an implantable pacemaker device, such as a leadless pacemaker device implanted within the patient's heart, is operative to conduct threshold test measurements in which a threshold value is determined for obtaining a so-called capture when injecting stimulation signals into cardiac tissue. During operation, the implantable pacemaker device outputs stimulation pulses to cause a pacing of the patient's heart. The stimulation pulses herein must have an amplitude and pulse width such that the stimulation pulses carry sufficient energy to effectively induce a stimulation of the conductive structure of the patient's heart, such that a stimulation pulse causes a desired pacing event, in particular an atrial or ventricular contraction event. If the amplitude and/or pulse width of the stimulation pulse is too small, a so-called capture loss may occur, in the case of which the stimulation pulse does not result in a subsequent pacing event.

To determine at what amplitude and/or pulse width a stimulation pulse should be output in order to reliably obtain a capture, a so-called threshold test measurement is performed by the implantable pacemaker device, in the course of which for example test pulses at varying amplitudes are output by the implantable pacemaker device and based on such test pulses the implantable pacemaker device determines a threshold value of the pulse amplitude (e.g. while assuming a fixed pulse width) at which a reliable stimulation may be achieved and below which no reliable stimulation is observed.

The implantable pacemaker device may for example be configured to conduct a threshold test measurement at regular intervals, for example once per day or twice the day. Herein, a threshold test measurement should preferably be conducted by the implantable pacemaker device when the patient is at rest and a steady, low heart rate is present. For example, a threshold test measurement hence may be performed by the implantable pacemaker device during the night when it is likely that the patient is asleep.

In that the implantable medical device, which may in particular be implanted extracardially, is configured to observe the implantable pacemaker device to obtain information about a threshold test measurement performed by the implantable pacemaker device and to communicate information concerning the threshold test measurement to an external device, the implantable medical device may establish a monitoring function for the implantable pacemaker device. Information relating to the operation of the implantable medical device, in particular relating to the conduction of a threshold test measurement, hence may be monitored by the implantable medical device and may be communicated by the implantable medical device to an external device. In this way data concerning the operation of the implantable pacemaker device for conducting the threshold test measurement is obtained using the implantable medical device and is communicated to the external device by means of the implantable medical device, hence making a direct data communication in between the implantable pacemaker device and the external device generally dispensable.

In principle, hence, the implantable pacemaker device does not need to establish a (direct) communication to the external device, such that the implantable pacemaker device may be operated in a particularly energy-efficient manner.

A monitoring of the operation of the implantable pacemaker device for conducting a threshold test measurement herein may reliably be established even if the implantable pacemaker device is implanted deep in the patient, hence making a direct data communication of the implantable pacemaker device to an external device potentially difficult. In that a monitoring of the implantable pacemaker device when conducting a threshold test measurement is carried out by the implantable medical device based on the sensing of electrocardiogram signals, no data transmission from the implantable pacemaker device to an external device is required to communicate information relating to a measured threshold for producing stimulation signals, such that a deep implantation state of the implantable pacemaker device does not impact data communication.

In that the external device receives information relating to a threshold test measurement conducted by the implantable pacemaker device, the external device may record and analyze results of threshold test measurements as conducted by the implantable pacemaker device. In that information relating to threshold test measurements as conducted by the implantable pacemaker device are obtained using the implantable medical device, which senses signals in relation to threshold test measurements performed by the implantable pacemaker device, no direct data communication in between the implantable pacemaker device and the implantable medical device and in between the implantable pacemaker device and the external device needs to be established, and no specific patient interaction for obtaining threshold test measurement results is required.

The implantable medical device is in one embodiment configured to identify stimulation events caused by the implantable pacemaker device based on sensed electrocardiogram signals. Within regular operation the implantable medical device records electrocardiogram signals and processes sensed electrocardiogram signals, wherein based on the processing stimulation events relating to a threshold test measurement as performed by the implantable pacemaker device may be identified and information relating to the stimulation events may be derived and transmitted to the external device. As the identification of the stimulation events relating to a threshold test measurement does not require a direct communication in between the implantable pacemaker device and the implantable medical device, requirements for a compatibility of the devices are negligible. A monitoring of an implantable pacemaker device hence may be established for example even in scenarios in which the implantable pacemaker device and the implantable medical device originate from different manufacturers.

In one embodiment, the processing circuitry is configured to store a portion of the sensed electrocardiogram signals relating to a threshold test measurement and to transmit a stored portion of the sensed electrocardiogram signals to the device external to the patient. If it is identified by the implantable medical device that the implantable pacemaker device conducts a threshold test measurement (e.g. if stimulation events are identified which are output by the implantable pacemaker device in the course of the conduction of a threshold test measurement by the implantable pacemaker device), signal portions relating to the threshold test measurement may be stored and may be communicated to the external device, such that signal portions indicative of the threshold test measurement are forwarded to the external device for further processing.

In one embodiment, the processing circuitry comprises a circular buffer for circularly storing sensed electrocardiogram signals. The circular buffer, also denoted as ring memory, may for example comprise a storage capacity for storing a signal portion of a specified time length, for example in a range between 10 seconds and 5 minutes, in particular in between 20 seconds and 1 minute. The circular buffer herein overwrites circularly its memory contents, such that the circular buffer at any time contains a signal portion of a defined prior time span.

In case it is identified that the implantable pacemaker device conducts a threshold test measurement, the processing circuitry may cause a transmission of a data message containing a multiplicity of stimulation events indicative of test pulses output during the threshold test measurement by the implantable pacemaker device. Resulting from the identification of a threshold test measurement, hence, a signal portion is extracted from the circular buffer and is forwarded to the external device, such that electrocardiogram signals indicative of the threshold test measurement are transmitted to the external device. The signal portion communicated to the external device in particular may encompass test pulses which are output by the implantable pacemaker device during the threshold test measurement to determine the stimulation threshold at which a reliable stimulation by a stimulation pulse may be achieved, below which however no reliable stimulation is observed.

Alternatively or in addition, the processing circuitry may be configured to analyze stimulation events indicative of test pulses output during the threshold test measurement by the implantable pacemaker device and to communicate information relating to an identified stimulation threshold to the device external to the patient. In particular, the processing circuitry may be configured, by processing sensed electrocardiogram signals indicative of stimulation events as induced by the implantable pacemaker device, to derive a value for the stimulation threshold as determined by the implantable pacemaker device during the threshold test measurement. The implantable medical device may then communicate the actual value for the stimulation threshold to the external device.

In one embodiment, the processing circuitry is configured to count stimulation events indicative of test pulses output during a threshold test measurement by the implantable pacemaker device to determine a stimulation threshold value as identified by the implantable pacemaker device in the course of the threshold test measurement. Once the processing circuitry has identified that the implantable pacemaker device is conducting a threshold test measurement, the processing circuitry may observe how many test pulses are output by the implantable pacemaker device. In particular, the implantable pacemaker device may be programmed to output a specified sequence of test pulses in order to determine the stimulation threshold, the specified sequence being generally known to the implantable medical device. Within the sequence, the implantable pacemaker device for example may start by outputting a test pulse at a defined start amplitude, and starting from the start amplitude it may successively reduce the amplitude of the test pulses by employing a defined scheme, which is known to the implantable medical device. Once the implantable pacemaker device finds that a test pulse at a particular amplitude does not yield an evoked response (e.g. a ventricular contraction subsequent to the emission of a test pulse), but rather results in a so-called capture loss, it may be assumed that a (larger) amplitude of a prior test pulse corresponds to the threshold value. As the scheme at which the implantable pacemaker device is programmed to vary the amplitudes of the test pulses is known to the implantable medical device, based on a counting of test pulses it may be determined how many test pulses are output by the implantable pacemaker device before the measurement is terminated by the pacemaker device, from which it may be concluded what the measured threshold is. This value may then be communicated to the external device by the implantable medical device.

In one embodiment, the processing circuitry is configured to evaluate a timing of stimulation events in order to identify at least one backup pulse output by the implantable pacemaker device subsequent to a prior test pulse and to draw conclusions, based on the occurrence of the at least one backup pulse, with respect to a stimulation threshold value as identified by the implantable pacemaker device in the course of the threshold test measurement. For example, during a threshold test measurement the implantable pacemaker device may produce output test pulses at progressively decreasing amplitudes. If for a test pulse no subsequent evoked response is detected, this may be identified as a capture loss by the pacemaker device, upon which the implantable pacemaker device may be programmed to output a so-called backup pulse in order to make sure that a steady pacing of the patient's heart is established. The backup pulse generally is output at a predefined timing distance, for example at a timing distance in between 50 ms to 150 ms, for example 100 ms, subsequent to a prior test pulse, such that based on observing a timing in between two subsequent stimulation events the processing circuitry of the implantable medical device may identify that a first stimulation event corresponds to a test pulse and a second stimulation event immediately following the first stimulation event at a predefined timing corresponds to a backup pulse. If the pacemaker device is programmed to output backup pulses only in case of a capture loss, it may be determined based on the occurrence of backup pulses if for a test pulse a capture loss is obtained at the pacemaker device, based on which information relating to the stimulation threshold as measured by the implantable pacemaker device may be derived.

In one embodiment, the processing circuitry is configured to identify a conduction of a threshold test measurement by the implantable male pacemaker device based on a recognition of a predefined pulse pattern output by the implantable pacemaker device during the conduction of the threshold test measurement. For example, the implantable pacemaker device may be programmed to output test pulses and/or backup pulses according to a predefined pattern during the conduction of a threshold test measurement, e.g. at a specific timing. The pattern may be known to the implantable medical device, such that by observing a particular pulse pattern output by the implantable pacemaker device the processing circuitry of the implantable medical device may identify that the implantable pacemaker device is conducting a threshold test measurement. Based on the identification, then, the processing circuitry may record electrocardiogram signals relating to the threshold test measurement as conducted by the implantable pacemaker device, or may process sensed signals in order to derive information relating to the threshold test measurement as conducted by the implantable pacemaker device.

In one embodiment, the implantable pacemaker device may be configured, at the start of a threshold test measurement, to output test pulses and/or backup pulses according to a predefined signal analysis sequence in a startup phase of the threshold test measurement. Within the signal analysis sequence, test pulses at at a comparatively large amplitude may be output according to a predefined pulse pattern in order to assess whether evoked responses are reliably obtained. Alternatively or in addition, pairs of pulses may be output by the implantable pacemaker device according to a predefined pulse pattern within the signal analysis sequence, each pair containing one test pulse and a subsequent backup pulse, the backup pulses serving to verify that a polarization artifact is small enough to distinguish capture from non-capture. Hence, the processing circuitry of the implantable medical device may be configured to identify a particular pulse pattern relating to a signal analysis sequence at the startup of a threshold test measurement in order to determine that the implantable pacemaker device is starting a threshold test measurement.

In one embodiment, the processing circuitry is configured to identify a conduction of a threshold test measurement by the implantable pacemaker device based on a programmed time at which the implantable pacemaker device is programmed to conduct the threshold test measurement. For example, the implantable pacemaker device may be programmed to regularly conduct a threshold test measurement at a predefined time, each day for example at 2 o'clock in the morning. This time may be known to the implantable medical device, such that the processing circuitry of the implantable medical device may be configured to record and/or process sensed signals at the predefined time corresponding to the time of conduction of the threshold test measurement in order to derive information relating to the threshold test measurement as conducted by the implantable pacemaker device.

In one embodiment, the processing circuitry is configured to trigger a data communication message to the device external to the patient based on the identification of the conduction of a threshold test measurement by the implantable pacemaker device. Hence, in an event-based approach a data communication may be initiated for example immediately upon identifying a threshold test measurement.

Alternatively or in addition, information concerning a threshold test measurement may be included in a data message which is transmitted by the implantable medical device according to a predefined, regular communication scheme. The implantable medical device may for example be configured to regularly send data messages to the external device. In such regular messages also information concerning the threshold test measurement may be included.

An implantable therapy system comprises, in one embodiment, an implantable pacemaker device for emitting a cardiac stimulation signal for achieving an intracardiac pacing, and an implantable medical device of the type described above. The implantable pacemaker device may for example be a leadless pacemaker device which is to be implanted directly into the heart, for example the right or left ventricle or the right or left atrium. The pacemaker device may for example provide for a therapy in case a bradycardia is detected in order to pace the patient's heart and to in this way pace the heart rate to a desired, normal heart rate. Alternatively or in addition, the pacemaker device may provide for a therapy in case a tachycardia is detected in order to reset the heart rate to a desired normal rate.

In another aspect, a method for operating an implantable medical device for providing a diagnostic and/or therapeutic cardiac function within a patient comprises: sensing, using a sensing arrangement of the implantable medical device, electrocardiogram signals; processing, using a processing circuitry of the implantable medical device, sensed electrocardiogram signals, wherein the processing includes: identifying the conduction of a threshold test measurement by an implantable pacemaker device and causing a transmission of information concerning said threshold test measurement to the device external to the patient.

The advantages and advantageous embodiments described above for the implantable medical device and the implantable therapy system equally apply also to the method, such that it shall be referred to the above in this respect.

The idea of the invention shall subsequently be described in more detail with reference to the embodiments as shown in the drawings. Herein:
- Fig. 1: shows a schematic drawing of a therapy system of medical devices implanted in a patient;
- Fig. 2: shows a schematic drawing of an implantable medical device together with an implantable pacemaker device;
- Fig. 3: shows a schematic drawing of a startup phase of a threshold test measurement as performed by an implantable pacemaker device; and
- Fig. 4: shows a schematic sequence of an example of a threshold test measurement as performed by an implantable pacemaker device.

Subsequently, embodiments of the invention shall be described in detail with reference to the drawings. In the drawings, like reference numerals designate like structural elements.

It is to be noted that the embodiments are not limiting for the invention, but merely represent illustrative examples.

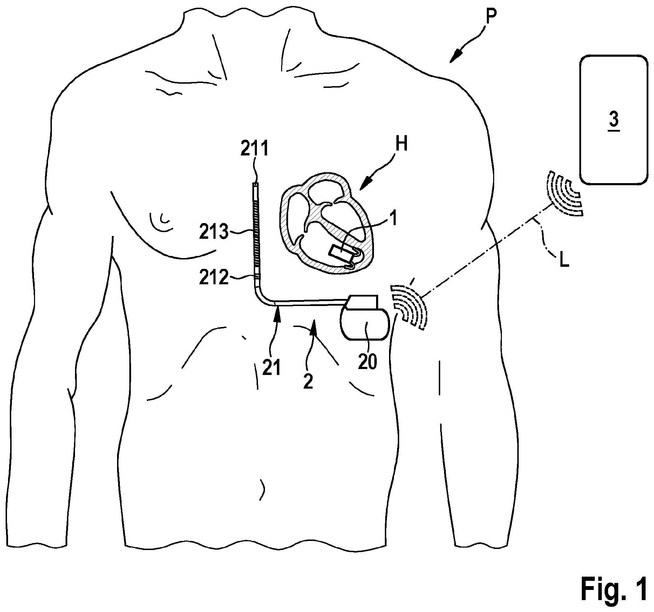

Referring to Fig. 1, in a setup of a therapy system an implantable leadless pacemaker device 1 is implanted in the heart H of a (human or animal) patient. The leadless pacemaker device 1 is implanted directly into the heart H, for example in the right ventricle RV, in order to couple to tissue within the heart H and to in this way sense cardiac signals within the heart and emit stimulation signals into cardiac tissue.

In addition, an implantable medical device 2 in the shape of a non-transvenous implantable defibrillator device is implanted such that the implantable medical device 2 is completely external to the heart H, the non-transvenous implantable medical device 2 comprising a generator 20 encapsulated within a housing, and a lead 21 connected to the generator 20 and carrying sensing electrodes 211, 212 as well as a shock electrode 213 in the shape of a coil formed on a distal portion of the lead 21. The sensing electrodes 211, 212, for example formed as ring electrodes on either side of the shock electrode 213, serve to sense cardiac signals for processing within the generator 20 of the implantable medical device 2, such that based on sensed signals e.g. a tachycardia may be identified and a shock pulse may be generated for providing for an anti-tachycardia therapy.

In a setup as shown in Fig. 1, the pacemaker device 1 implanted within the heart H and the implantable medical device 2 implanted outside of the heart H each comprise sensing circuitry, such that based on a processing of sensed signals in the respective device 1, 2 a therapeutic action may be initiated. Each device 1, 2 herein functions, in principle, independent of the other device 2, 1, such that by means of the pacemaker device 1 implanted in the heart H stimulation signals e.g. for overcoming a bradycardia may be generated, whereas by means of the defibrillator device 2 a shock pulse may be emitted for resetting e.g. a tachycardic heart rate to a normal heart rate.

In a setup as shown in Fig. 1, the implantable pacemaker device 1 is implanted in the heart H and hence rather deep within the patient P. The implantable medical device 2, in contrast, with its generator 20 may for example be implanted subcutaneously, the lead 21 extending from the generator 20 such that it extends outside of the heart H and hence does not reach transvenously into the heart H. The implantable medical device 2 is configured to establish a communication link L to an external device 3 such that a data communication in between the implantable medical device 2 and the external device 3 may take place.

The external device 3 may for example be part of a home monitoring system. The external device 3 for example resides at the site, e.g. at the home, of the patient P and beneficially is in connection with a home monitoring service center, such that data may be relayed to and from the home monitoring center by means of the external device 3.

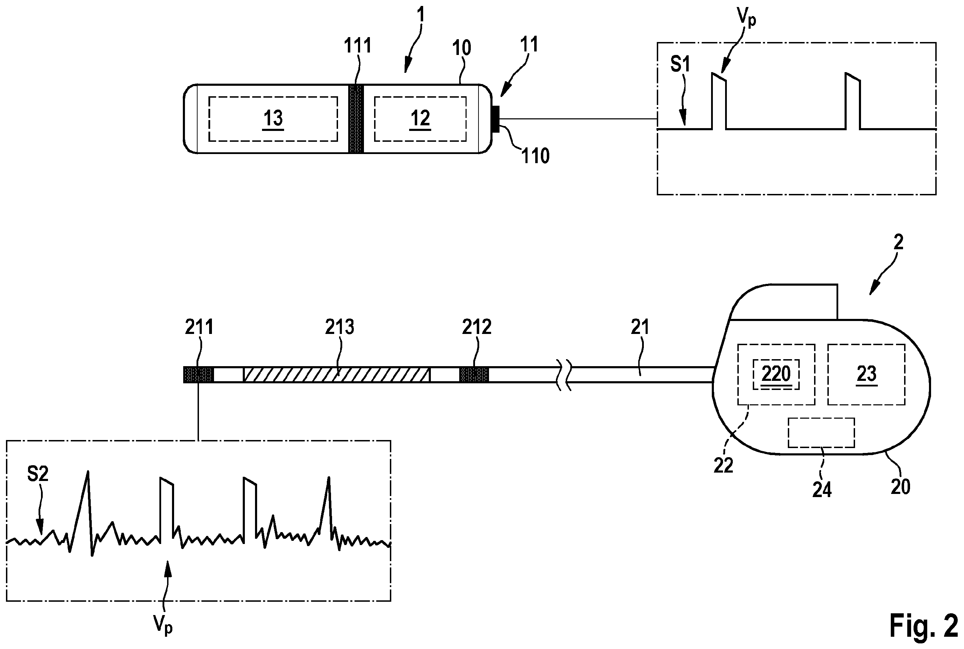

Referring now to Fig. 2, the pacemaker device 1 comprises a housing 10 on which an electrode arrangement 11 having electrode poles 110, 111 is arranged. The electrode poles 110, 111 form a pair of electrodes by means of which stimulation pulses may be injected into tissue and sense signals may be received from tissue to provide for a stimulation and sensing at the implantation site of the implantable pacemaker device 1. The implantable pacemaker device 1 furthermore comprises control circuitry 12 and a battery 13 providing for an energy storage, both the control circuitry 12 and the battery 13 being encapsulated in the housing 10.

The non-transvenous implantable medical device 2, within the generator 20, comprises processing circuitry 22, a battery 23 providing for an energy storage, and communication circuitry 24.

The processing circuitry 22 serves to process signals received via the sensing arrangement of the sensing electrodes 211, 212 and to trigger a stimulation action by emitting a shock pulse via the shock electrode 213 in case e.g. a tachycardia is detected.

By means of the communication circuitry 24 a communication link L (see Fig. 1) to the external device 3 is established, the communication circuitry 24 being configured for example to establish a communication according to the MICS protocol, the ISM protocol, the BLE protocol, the Zigbee protocol, or according to another communication scheme.

The pacemaker device 1 and the non-transvenous implantable medical device 2, in the shown embodiment, are not functioning in immediate connection with each other. That is, the pacemaker device 1 and the implantable medical device 2 are not in data connection with each other, such that no control signals are exchanged in between the pacemaker device 1 and the implantable medical device 2.

Rather, the pacemaker device 1 functions, for example, without knowledge about the presence of the implantable medical device 2 by sensing electrocardiogram signals within the heart H and by emitting stimulation signals S1 based on a processing of sensed signals. The pacemaker device 1, for example, may be configured to detect a bradycardia, and to emit stimulation pulses to cause stimulation events Vp in case a bradycardia is detected.

In the example of Figs. 1 and 2, the pacemaker device 1 does not directly communicate with the external device 3. In order to nevertheless provide for a monitoring of the operation of the pacemaker device 1, the implantable medical device 2 is configured to identify, within sensed electrocardiogram signals S2, stimulation events Vp caused by the pacemaker device 1.

For example, the sensed electrocardiogram signals S2 may be processed within the processing circuitry 22 of the generator 20 of the implantable medical device 2 by applying e.g. a digitalization, an amplification and a filtering in order to discern a stimulation pulse of the implantable pacemaker device 1 from intrinsic electrocardiogram signals originating from cardiac activity. For example, the processing circuitry 22 may process sensed electrocardiogram signals S2 by assessing a positive or negative flank of a pulse, for example by evaluating the flank's gradient or the flank' height, or by applying pattern recognition techniques in order to identify a predefined pulse shape stemming from a stimulation pulse as emitted by the pacemaker device 1.

In one embodiment, the processing circuitry 22 comprises a circular buffer 220 which serves to circularly store sensed electrocardiogram signals S2 such that a portion of the sensed electrocardiogram signals S2 relating to a prior time span is always kept in memory and is circularly overwritten by new data. For example, the circular buffer 220 may be configured to keep a signal portion for a time span between 10 seconds and 5 minutes, for example between 20 seconds and 1 minute, in memory such that always a most recent signal portion is stored within the circular buffer 220.

The processing circuitry 22 is configured to derive information from the sensed electrocardiogram signals S2 in relation to the operation of the pacemaker device 1. The processing circuitry 22 hence is operative to enable a monitoring of the operation of the pacemaker device 1 based solely on the reception of electrocardiogram signals S2 and by assessing the action of the pacemaker device 1 according to sensed electrocardiogram signals S2. Based on the monitoring of the action of the pacemaker device 1, data may be transmitted to the external device 3 in order to provide for a monitoring of the operation of the pacemaker device 1.

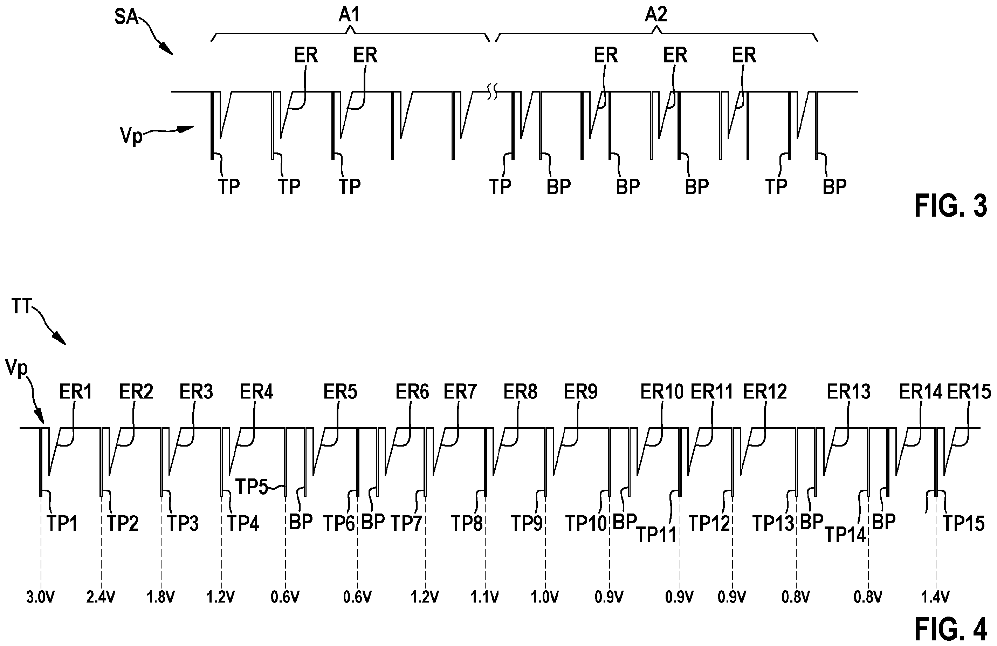

Specifically, referring now to Figs. 3 and 4, the implantable pacemaker device 1 is configured to perform a threshold test measurement TT, an example of which is shown in Fig. 4, in order to determine a stimulation threshold value of a pulse amplitude for a stimulation pulse such that a stimulation pulse with an amplitude above the threshold value reliably induces a cardiac stimulation, in particular a ventricular contraction, a stimulation pulse having an amplitude below the threshold value however not reliably causing a cardiac stimulation.

For example, the implantable pacemaker device 1 may be configured to conduct threshold test measurements TT regularly once a day or twice per day, for example at a programmed time at which the patient P can be assumed to be at rest, for example during the night, for example each day at e.g. 2 a.m. In the course of a threshold test measurement TT test pulses TP are produced and output by the implantable pacemaker device 1, and evoked responses ER are observed in order to determine whether a test pulse TP at a specific amplitude induces a contraction and hence corresponds to a capture.

Within the setup of Fig. 1, the implantable medical device 2 is configured to observe stimulation events Vp corresponding to test pulses TP and/or so-called backup pulses BP as output by the implantable pacemaker device 1 when conducting a threshold test measurement TT. When the implantable medical device 2 identifies that the pacemaker device 1 currently conducts a threshold test measurement TT, the processing circuitry 22 may store signal portions relating to the threshold test measurement TT in the circular buffer 220 and may communicate such signal portions to the external device 3. Alternatively or in addition, the processing circuitry 22 may process the electrocardiogram signals S2 as sensed during a threshold test measurement TT as performed by the implantable pacemaker device 1 and may derive, from stimulation events Vp corresponding to test pulses TP and/or backup pulses BP in the course of the threshold test measurement TT, information relating to the threshold test measurement TT as carried out by the implantable pacemaker device 1.

The implantable medical device 2 may for example determine that the implantable pacemaker device 1 is carrying out a threshold test measurement TT based on a programmed time at which the implantable pacemaker device 1 is programmed to conduct a threshold test measurement TT and which is known also to the implantable medical device 2. If for example the implantable pacemaker device 1 is programmed to conduct a threshold test measurement each day at 2 a.m. in the morning, the implantable medical device 2 may sense signals at the programmed time and may record and/or process sensed signals in order to communicate information relating to the threshold test measurement TT to the external device 3.

In another embodiment, the implantable medical device 2 may determine that the implantable pacemaker device 1 is carrying out a threshold test measurement TT based on identifying a predefined signal pattern relating to the threshold test measurement TT, for example a signal pattern relating to a startup phase of the threshold test measurement TT at which the implantable pacemaker device 1 outputs a signal analysis sequence SA as schematically shown in Fig. 3.

In a startup phase prior to conducting the actual threshold test measurement TT, the implantable pacemaker device 1 may for example output a predefined pattern of test pulses TP and backup pulses BP in order to assess whether the threshold test measurement TT should be conducted or not.

During the signal analysis sequence SA, the implantable pacemaker device 1 may for example, in a first phase A1, output a sequence of test pulses TP at a predefined signal amplitude, for example corresponding to a programmed start value which should reliably yield an evoked response ER, in order to assess whether the test pulses TP in fact yield evoked responses ER corresponding to cardiac contractions induced by the test pulses TP. In a subsequent, second phase A2 the implantable pacemaker device 1 outputs pairs of pulses, each pair comprising a test pulse TP and a backup pulse BP, the backup pulse BP following the test pulse TP at a predefined timing distance, for example 100 ms. The test pulses TP in the second phase A2 may have a pulse amplitude corresponding to the amplitude of the test pulses TP during the first phase A1, such that evoked responses ER are reliably induced by the test pulses TP. The backup pulses BP in the second phase A2 serve to assess a so-called polarization artifact, in particular to ensure that the polarization artifact is small enough to distinguish a capture from a capture loss.

The pulse pattern or a portion of the pulse pattern as shown in Fig. 3 and as output as an initial signal analysis sequence SA in the startup phase of the threshold test measurement TT may be observed and identified by the implantable medical device 2 based on a processing of electrocardiogram signals S2. In particular, in the electrocardiogram signals S2 the processing circuitry 22 of the implantable medical device 2 may identify stimulation events Vp corresponding to the test pulses TP and/or to the pairs of pulses TP, BP as output during the different phases A1, A2 of the initial startup phase. If a particular pattern is identified according to pulses in the sensed electrocardiogram signal S2, the implantable medical device 2 may conclude that the implantable pacemaker device 1 is about to start a threshold test measurement TT and may hence start recording electrocardiogram signals S2 relating to a subsequent threshold test measurement TT.

Referring now to Fig. 4, during an actual threshold test measurement TT the implantable pacemaker device 1 produces test pulses TP at progressively decreasing pulse amplitudes in order to assess, based on evoked responses ER, whether a test pulse TP (still) yields a capture.

In the example of Fig. 4, an initial test pulse TP1 may for example be produced by the implantable pacemaker device 1 at a start amplitude value of 3.0 V. A subsequent evoked response ER1 is sensed by the implantable pacemaker device 1 indicating a capture, upon which a second test pulse TP2 at a reduced amplitude of 2.4 V is produced, upon which again an evoked response ER2 is sensed. The pulse amplitude now is progressively reduced in steps of 0.6 V (test pulse TP3 at 1.8 V, yielding evoked response ER3; test pulse TP4 at 1.2 V, yielding evoked response ER4; test pulse TP5 at 0.6 V), until no evoked response is observed by the implantable pacemaker device 1 following test pulse TP5.

In order to ensure that during the threshold test measurement a steady pacing of the patient is obtained, subsequent to test pulse TP5 a backup pulse BP is output at a predefined timing distance, for example 100 ms, and at a predefined pulse amplitude, corresponding for example to the start amplitude value of the test pulse TP1, such that a contraction is induced.

Subsequently, another test pulse TP6 at the same amplitude (0.6 V) as for the test pulse TP5 is output, upon which again no evoked response is sensed, and another backup pulse BP is output.

The test pulse TP6 at the same amplitude as the test pulse TP5 is output in order to assess whether a capture loss for test pulse TP5 potentially has been erroneous. As also the test pulse TP6 does not yield an evoked response, the pulse amplitude is now increased back to the value of the last successful test pulse TP4, and a test pulse TP7 at 1.2 V is output, upon which an evoked response ER7 is sensed. The pulse amplitude is now progressively reduced by steps of 0.1 V, such that test pulses TP8, TP9, TP10 at amplitudes 1.1 V, 1.0 V, 0.9 V, respectively, are produced. Whereas the test pulses TP8, TP9 induce evoked responses ER8, ER9 and hence each result in a capture, the test pulse TP10 does not yield an evoked response, upon which a backup pulse BP is output. Subsequently, further test pulses TP11, TP12 at 0.9 V are produced and output by the implantable pacemaker device 1, each test pulse TP11, TP12 inducing an evoked response ER11, ER12, such that it is assumed that a pulse amplitude of 0.9 V still yields a reliable capture (a test pulse at a specific amplitude is repeated at most three times, and if two out of the three test pulses yield an evoked response, it is assumed that a capture is obtained).

Now, a test pulse TP13 at a reduced amplitude of 0.8 V is output, followed by a backup pulse BP, as the test pulse TP13 does not yield an evoked response. As also another test pulse TP14 at 0.8 V does not yield an evoked response (and hence is followed by a backup pulse BP), it is assumed that the threshold value lies at 0.9 V, corresponding to the amplitude of the test pulses TP10-TP12, which still have resulted in a successful capture.

According to the threshold value determined in this way, then, the amplitude for stimulation pulses for subsequent device operation is set according to the threshold value plus a predefined safety margin, in the shown example equal to 0.5 V, the amplitude of the stimulation pulses during subsequent operation of the pacemaker device 1 hence corresponding to 1.4 V, which is verified by a test pulse TP15 and results in a corresponding evoked response ER15.

The implantable medical device 2, according to sensed electrocardiogram signals S2, observes and monitors a pulse sequence corresponding to the threshold test measurement TT as shown in an example in Fig. 4. The implantable medical device 2 may record a portion of the electrocardiogram signals S2 corresponding to the threshold test measurement TT and may communicate such signal portion to the external device 3.

Alternatively or in addition, the implantable medical device 2 may process electrocardiogram signals S2 as sensed in the context of a threshold test measurement TT as performed by the implantable pacemaker device 1. For example, the implantable medical device 2 may process the electrocardiogram signals S2 in order to determine stimulation events Vp in the electrocardiogram signals S2 relating to test pulses TP1 ... TP15 and to derive the threshold value as determined by the implantable pacemaker device 1 during the threshold test measurement TT.

For the processing, the processing circuitry 22 of the implantable medical device 2 may for example be configured to count test pulses TP1... TP15. As the specific scheme according to which the implantable pacemaker device 1 varies the pulse amplitude of the test pulses TP1...TP15 is known to the implantable medical device 2, by counting the test pulses TP1... TP15 it may be identified at which test pulse the measurement stops, and from this it may be derived what the measured threshold value is.

Herein, the implantable medical device 2 in addition may identify backup pulses BP as output by the implantable pacemaker device 1 following test pulses TP which do not yield an evoked response. As during the threshold test measurement TT backup pulses BP are output only if a test pulse TP does not yield an evoked response, based on a backup pulse BP it may be determined when the implantable pacemaker device 1 changes from a 0.6 V step change in the test pulses to a 0.1 V step change and when the test sequence ends.

In one embodiment, the processing circuitry 22 is configured to cause a transmission of information to the external device 3 based on the identification of a threshold test measurement TT. For example, in case a threshold test measurement TT is carried out by the implantable pacemaker device 1 and is correspondingly identified within the electrocardiogram signal S2 by the implantable medical device 1 or according to a programmed time of execution, a signal portion containing stimulation events Vp corresponding to the threshold test measurement TT and test pulses and backup pulses output therein may be transmitted within a data message to the external device 3, such that the external device 3 obtains information about the threshold test measurement TT as performed by the pacemaker device 1.

Based on an identification of a threshold test measurement TT, the implantable medical device 2 may cause a data communication with the external device 3.

In an event-based communication, for example, the implantable medical device 2 may for example trigger a communication message on the event of identifying a threshold test measurement as performed by the pacemaker device 1. Based on the detection of the event, hence, a data message is created and transmitted to the external device 3 using the communication circuitry 24.

In addition or alternatively, information concerning the pacemaker device 1 may be included in data messages as transmitted to the external device 3 according to a regular, predefined communication scheme, within which for example data messages are exchanged in between the implantable medical device 2 and the external device 3 in a regular, prescheduled manner. The identification of a threshold test measurement as performed by the pacemaker device 1 thus for example does not cause an immediate triggering of a specific, dedicated communication message, but information relating to the operation of the pacemaker device 1 is included in a regular communication message, for example to provide for a periodic reporting.

The implantable medical device may in particular be an implantable cardioverter defibrillator device (ICD). In another embodiment, the implantable medical device may be a monitoring device, such as a so-called biomonitor, to be implanted e.g. subcutaneously in proximity to the heart for sensing and monitoring signals, which however does not by itself provide for a therapeutic function by emitting stimulation signals.

### List of reference numerals

- 1: Pacemaker device
- 10: Housing
- 11: Electrode arrangement
- 110, 111: Electrode poles
- 12: Control circuitry
- 13: Energy storage (battery)
- 2: Non-transvenous defibrillator device
- 20: Generator
- 21: Lead
- 211, 212: Sensing electrode
- 213: Shock electrode
- 22: Processing circuitry
- 220: Circular buffer
- 23: Energy storage (battery)
- 24: Communication circuitry
- 3: External device
- A1, A2: Phase
- BP: Backup pulse
- ER, ER1..ER13: Evoked response
- H: Heart
- L: Communication link
- P: Patient
- SA: Signal analysis sequence
- S1: Stimulation signal
- S2: Sensed signal
- TP, TP1... TP13: Test pulse
- TT: Threshold test measurement
- Vp: Stimulation event

## Claims

1. An implantable medical device (2) for providing a diagnostic and/or therapeutic cardiac function within a patient (P), comprising:
a sensing arrangement for sensing electrocardiogram signals;
a communication circuitry (24) for data communication with a device (3) external to the patient (P); and
a processing circuitry (22) for processing sensed electrocardiogram signals (S2), wherein the processing circuitry (22) is configured to identify the conduction of a threshold test measurement (TT) by an implantable pacemaker device (1) and to cause a transmission of information concerning said threshold test measurement (TT) to the device (3) external to the patient (P),
wherein the processing circuitry (22) is configured to analyze a multiplicity of stimulation events (Vp) indicative of test pulses (TP) output during the threshold test measurement by the implantable pacemaker device (1) and to communicate information relating to an identified stimulation threshold to the device (3) external to the patient (P).

2. The implantable medical device (2) according to claim 1, **characterized in that** the implantable medical device (2) is an implantable non-transvenous defibrillator device configured to emit a shock pulse for achieving a defibrillation.

3. The implantable medical device (2) according to claim 2, **characterized in that** the implantable non-transvenous defibrillator device comprises a lead (21) to be implanted extracardially and a shock electrode (213) arranged on the lead (21).

4. The implantable medical device (2) according to one of claims 1 to 3, **characterized in that** the processing circuitry (22) is configured to store a portion of the sensed electrocardiogram signals (S2) relating to said threshold test measurement (TT) and to transmit a stored portion of the sensed electrocardiogram signals (S2) to the device (3) external to the patient (P).

5. The implantable medical device (2) according to claim 4, **characterized in that** the processing circuitry (22) comprises a circular buffer (220) for circularly storing the sensed electrocardiogram signals (S2).

6. The implantable medical device (2) according to claim 4 or 5, **characterized in that** said portion comprises a signal portion containing a multiplicity of stimulation events (Vp) indicative of test pulses (TP) output during the threshold test measurement by the implantable pacemaker device (1).

7. The implantable medical device (2) according to one of the preceding claims, **characterized in that** the processing circuitry (22) is configured to count stimulation events (Vp) indicative of test pulses (TP) output during the threshold test measurement by the implantable pacemaker device (1) to determine a stimulation threshold value as identified by the implantable pacemaker device (1) in the course of the threshold test measurement (TT).

8. The implantable medical device (2) according to one of the preceding claims, **characterized in that** the processing circuitry (22) is configured to evaluate a timing of stimulation events (Vp) in order to identify at least one backup pulse (BP) output by the implantable pacemaker device (1) subsequent to a prior test pulse (TP) and to determine, based on the occurrence of the at least one backup pulse (BP), a stimulation threshold value as identified by the implantable pacemaker device (1) in the course of the threshold test measurement (TT).

9. The implantable medical device (2) according to one of the preceding claims, **characterized in that** the processing circuitry (22) is configured to identify a conduction of a threshold test measurement (TT) by the implantable pacemaker device (1) based on a recognition of a predefined pulse pattern output by the implantable pacemaker device (1) during the conduction of the threshold test measurement (TT).

10. The implantable medical device (2) according to one of the preceding claims, **characterized in that** the processing circuitry (22) is configured to identify a conduction of a threshold test measurement (TT) by the implantable pacemaker device (1) based on a recognition of a predefined pulse pattern of pulses output by the implantable pacemaker device (1) in the course of a signal analysis sequence (SA) in a startup phase of the threshold test measurement (TT).

11. The implantable medical device (2) according to one of the preceding claims, **characterized in that** the processing circuitry (22) is configured to identify a conduction of a threshold test measurement (TT) by the implantable pacemaker device (1) based on a programmed time at which the implantable pacemaker device (1) is programmed to conduct the threshold test measurement (TT).

12. An implantable therapy system, comprising: an implantable pacemaker device (1) for emitting a cardiac stimulation signal for achieving an intra-cardiac pacing; and an implantable medical device (2) according to one of the preceding claims.

13. The implantable therapy system according to claim 12, wherein the implantable pacemaker device (1) is a leadless pacemaker device.

## Patentansprüche

1. Implantierbare medizinische Vorrichtung (2) zum Bereitstellen einer diagnostischen und/oder therapeutischen Herzfunktion in einem Patienten (P), umfassend:
eine Erfassungsanordnung zum Erfassen von Elektrokardiogrammsignalen
eine Kommunikationsschaltung (24) zur Datenkommunikation mit einer Vorrichtung (3), die sich außerhalb des Patienten (P) befindet; und
eine Verarbeitungsschaltung (22) zum Verarbeiten erfasster Elektrokardiogrammsignale (S2), wobei die Verarbeitungsschaltung (22) konfiguriert ist, um das Durchführen einer Schwellenwert-Testmessung (TT) durch eine implantierbare Schrittmachervorrichtung (1) zu identifizieren und eine Übertragung von Informationen bezüglich der Schwellenwert-Testmessung (TT) an die Vorrichtung (3), die sich außerhalb des Patienten (P) befindet, zu veranlassen,
wobei die Verarbeitungsschaltung (22) konfiguriert ist, um eine Vielzahl von Stimulationsereignissen (Vp) zu analysieren, die auf Testimpulse (TP) hinweisen, die während der Schwellenwert-Testmessung durch die implantierbare Schrittmachervorrichtung (1) ausgegeben werden, und um Information in Bezug auf einen identifizierten Stimulations-Schwellenwert an die Vorrichtung (3), die sich außerhalb des Patienten (P) befindet, zu übermitteln.

2. Implantierbare medizinische Vorrichtung (2) nach Anspruch 1, **dadurch gekennzeichnet, dass** es sich bei der implantierbaren medizinischen Vorrichtung (2) um eine implantierbare nicht-transvenöse Defibrillatorvorrichtung handelt, die konfiguriert ist, um einen Schockimpuls zum Erreichen einer Defibrillation abzugeben.

3. Implantierbare medizinische Vorrichtung (2) nach Anspruch 2, **dadurch gekennzeichnet, dass** die implantierbare nicht-transvenöse Defibrillatorvorrichtung eine extrakardial zu implantierende Zuleitung (21) und eine an der Zuleitung (21) angeordnete Schockelektrode (213) umfasst.

4. Implantierbare medizinische Vorrichtung (2) nach Anspruch 1 bis 3, **dadurch gekennzeichnet, dass** die Verarbeitungsschaltung (22) konfiguriert ist, um einen auf die Schwellenwert-Testmessung (TT) bezogenen Abschnitt der erfassten Elektrokardiogrammsignale (S2) zu speichern und um einen gespeicherten Abschnitt der erfassten Elektrokardiogrammsignale (S2) an die Vorrichtung (3), die sich außerhalb des Patienten (P) befindet, zu übertragen.

5. Implantierbare medizinische Vorrichtung (2) nach Anspruch 4, **dadurch gekennzeichnet, dass** die Verarbeitungsschaltung (22) einen Ringpuffer (220) zum zirkulären Speichern der erfassten Elektrokardiogrammsignale (S2) umfasst.

6. Implantierbare medizinische Vorrichtung (2) nach Anspruch 4 oder 5, **dadurch gekennzeichnet, dass** der Abschnitt einen Signalabschnitt umfasst, der eine Vielzahl von Stimulationsereignissen (Vp) enthält, die auf Testimpulse (TP) hinweisen, die während der Schwellenwert-Testmessung durch die implantierbare Schrittmachervorrichtung (1) ausgegeben werden.

7. Implantierbare medizinische Vorrichtung (2) nach einem der vorhergehenden Ansprüche, **dadurch gekennzeichnet, dass** die Verarbeitungsschaltung (22) konfiguriert ist, um Stimulationsereignisse (Vp) zu zählen, die auf Testimpulse (TP) hinweisen, die während der Schwellenwert-Testmessung durch die implantierbare Schrittmachervorrichtung (1) ausgegeben werden, um einen Stimulationsschwellenwert zu bestimmen, den die implantierbare Schrittmachervorrichtung (1) im Verlauf der Schwellenwert-Testmessung (TT) identifiziert hat.

8. Implantierbare medizinische Vorrichtung (2) nach einem der vorhergehenden Ansprüche, **dadurch gekennzeichnet, dass** die Verarbeitungsschaltung (22) konfiguriert ist, eine Zeitsteuerung von Stimulationsereignissen (Vp) auszuwerten, um zumindest einen von der implantierbaren Schrittmachervorrichtung (1) im Anschluss an einen vorherigen Testimpuls (TP) ausgegebenen Backup-Impuls (BP) zu identifizieren und basierend auf dem Auftreten des zumindest einen Backup-Impulses (BP) einen Stimulationsschwellenwert zu bestimmen, den die implantierbare Schrittmachervorrichtung (1) im Verlauf der Schwellenwert-Testmessung (TT) identifiziert hat.

9. Implantierbare medizinische Vorrichtung (2) nach einem der vorhergehenden Ansprüche, **dadurch gekennzeichnet, dass** die Verarbeitungsschaltung (22) konfiguriert ist, um ein Durchführen einer Schwellenwert-Testmessung (TT) durch die implantierbare Schrittmachervorrichtung (1) basierend auf einer Erkennung eines vordefinierten Impulsmusters, das durch die implantierbare Schrittmachervorrichtung (1) während des Durchführens der Schwellenwert-Testmessung (TT) ausgegeben wird, zu identifizieren.

10. Implantierbare medizinische Vorrichtung (2) nach einem der vorhergehenden Ansprüche, **dadurch gekennzeichnet, dass** die Verarbeitungsschaltung (22) konfiguriert ist, um ein Durchführen einer Schwellenwert-Testmessung (TT) durch die implantierbare Schrittmachervorrichtung (1) basierend auf einer Erkennung eines vordefinierten Impulsmusters von Impulsen, die durch die implantierbare Schrittmachervorrichtung (1) im Verlauf einer Signalanalysesequenz (SA) in einer Startphase der Schwellenwert-Testmessung (TT) ausgegeben werden, zu identifizieren.

11. Implantierbare medizinische Vorrichtung (2) nach einem der vorhergehenden Ansprüche, **dadurch gekennzeichnet, dass** die Verarbeitungsschaltung (22) konfiguriert ist, das Durchführen einer Schwellenwert-Testmessung (TT) durch die implantierbare Schrittmachervorrichtung (1) basierend auf einem programmierten Zeitpunkt zu identifizieren, zu dem die implantierbare Schrittmachervorrichtung (1) programmiert ist, die Schwellenwert-Testmessung (TT) durchzuführen.

12. Implantierbares Therapiesystem, umfassend: eine implantierbare Schrittmachervorrichtung (1) zum Abgeben eines Herzstimulationssignals zum Erreichen einer intrakardialen Stimulation; und eine implantierbare medizinische Vorrichtung (2) nach einem der vorhergehenden Ansprüche.

13. Implantierbares Therapiesystem nach Anspruch 12, wobei es sich bei der implantierbaren Schrittmachervorrichtung (1) um eine kabellose Schrittmachervorrichtung handelt.

## Revendications

1. Dispositif médical implantable (2) destiné à fournir une fonction cardiaque diagnostique et/ou thérapeutique au sein d'un patient (P), comprenant :
un agencement de détection destiné à détecter des signaux d'électrocardiogramme ;
un circuit de communication (24) destiné à la communication de données avec un dispositif (3) externe au patient (P) ; et
un circuit de traitement (22) destiné au traitement de signaux d'électrocardiogramme (S2) détectés, dans lequel le circuit de traitement (22) est configuré pour identifier la conduction d'une mesure de test de seuil (TT) par un dispositif de stimulateur cardiaque implantable (1) et pour amener une transmission d'informations concernant ladite mesure de test de seuil (TT) au dispositif (3) externe au patient (P),
dans lequel le circuit de traitement (22) est configuré pour analyser une multiplicité d'événements de stimulation (Vp) indiquant la sortie d'impulsions de test (Tp) pendant la mesure de test de seuil par le dispositif de stimulateur cardiaque implantable (1) et pour communiquer des informations liées à un seuil de stimulation identifié au dispositif (3) externe au patient (P).

2. Dispositif médical implantable (2) selon la revendication 1, **caractérisé en ce que** le dispositif médical implantable (2) est un dispositif de défibrillateur non transveineux implantable configuré pour émettre une impulsion de choc afin d'obtenir une défibrillation.

3. Dispositif médical implantable (2) selon la revendication 2, **caractérisé en ce que** le dispositif de défibrillateur non transveineux implantable comprend un câble (21) à implanter de manière extracardiaque et une électrode de choc (213) agencée sur le câble (21).

4. Dispositif médical implantable (2) selon l'une des revendications 1 à 3, **caractérisé en ce que** le circuit de traitement (22) est configuré pour stocker une partie des signaux d'électrocardiogramme (S2) détectés liés à ladite mesure de test de seuil (TT) et pour transmettre une partie stockée des signaux d'électrocardiogramme (S2) détectés au dispositif (3) externe au patient (P).

5. Dispositif médical implantable (2) selon la revendication 4, **caractérisé en ce que** le circuit de traitement (22) comprend un tampon circulaire (220) pour stocker de manière circulaire les signaux d'électrocardiogramme (S2) détectés.

6. Dispositif médical implantable (2) selon la revendication 4 ou 5, **caractérisé en ce que** ladite partie comprend une partie de signal contenant une multiplicité d'événements de stimulation (Vp) indiquant une sortie d'impulsions de tests (TP) pendant la mesure de test de seuil par le dispositif de stimulateur cardiaque implantable (1).

7. Dispositif médical implantable (2) selon l'une des revendications précédentes, **caractérisé en ce que** le circuit de traitement (22) est configuré pour compter les événements de stimulation (Vp) indiquant une sortie d'impulsions de test (TP) pendant la mesure de test de seuil par le dispositif de stimulateur cardiaque implantable (1) afin de déterminer une valeur de seuil de stimulation comme identifié par le dispositif de stimulateur cardiaque implantable (1) au cours de la mesure de test de seuil (TT).

8. Dispositif médical implantable (2) selon l'une des revendications précédentes, **caractérisé en ce que** le circuit de traitement (22) est configuré pour évaluer une chronologie des événements de stimulation (Vp) afin d'identifier au moins une sortie d'impulsion de secours (BP) par le dispositif de stimulateur cardiaque implantable (1) après une impulsion de test (TP) antérieure et afin de déterminer, en se basant sur la survenue d'au moins une impulsion de secours (BP), une valeur de seuil de stimulation comme identifié par le dispositif de stimulateur cardiaque implantable (1) au cours de la mesure de test de seuil (TT).

9. Dispositif médical implantable (2) selon l'une des revendications précédentes, **caractérisé en ce que** le circuit de traitement (22) est configuré pour identifier une conduction d'une mesure de test de seuil (TT) par le dispositif de stimulateur cardiaque implantable (1) en se basant sur une reconnaissance d'une sortie de motif d'impulsion prédéfini par le dispositif de stimulateur cardiaque implantable (1) pendant la conduction de la mesure de test de seuil (TT).

10. Dispositif médical implantable (2) selon l'une des revendications précédentes, **caractérisé en ce que** le circuit de traitement (22) est configuré pour identifier une conduction d'une mesure de test de seuil (TT) par le dispositif de stimulateur cardiaque implantable (1) en se basant sur une reconnaissance d'un motif d'impulsion prédéfini d'une sortie d'impulsions par le dispositif de stimulateur cardiaque implantable (1) au cours d'une séquence d'analyse de signal (SA) dans une phase de démarrage de la mesure de test de seuil (TT).

11. Dispositif médical implantable (2) selon l'une des revendications précédentes, **caractérisé en ce que** le circuit de traitement (22) est configuré pour identifier une conduction d'une mesure de test de seuil (TT) par le dispositif de stimulateur cardiaque implantable (1) en se basant sur un temps programmé auquel le dispositif de stimulateur cardiaque implantable (1) est programmé pour mener la mesure de test de seuil (TT).

12. Système thérapeutique implantable, comprenant : un dispositif de stimulateur cardiaque implantable (1) destiné à émettre un signal de stimulation cardiaque pour obtenir une stimulation intracardiaque ; et un dispositif médical implantable (2) selon l'une des revendications précédentes.

13. Système thérapeutique implantable selon la revendication 12, dans lequel le dispositif de stimulateur cardiaque implantable (1) est un dispositif de stimulateur cardiaque sans câbles.
